# EUROPEAN PATENT SPECIFICATION

(11) **EP 2 378 890 B1**
(45) Date of publication and mention of the grant of the patent: **01.10.2014**
(21) Application number: 09760869.9
(22) Date of filing: 27.11.2009
(51) Int. Cl.: A23C 9/12, A23L 1/30, A61K 35/74, C12N 1/20

(54) **PREVENTION AND TREATMENT OF ROTAVIRUS DIARRHOEA**
PRÄVENTION UND BEHANDLUNG DER ROTAVIRUS-DIARRHÖE
PRÉVENTION ET TRAITEMENT D'UNE DIARRHÉE À ROTAVIRUS

(30) Priority: 19.12.2008 EP 08172263
(43) Date of publication of application: 26.10.2011
(73) Proprietor: Nestec S.A., 1800 Vevey (CH)
(72) Inventor: ARIGONI, Fabrizio, Tokyo 102-0084 (JP); BRUESSOW, Harald, CH-1814 La Tour-de-peilz (CH); CAVADINI, Christoph, CH-1804 Corsier-sur-Vevey (CH); PAGE, Nicolas, CH-1012 Lausanne (CH)
(74) Representative: Corticchiato, Olivier
(86) International application number: PCT/EP2009/065977
(87) International publication number: WO 2010/069737

(56) References cited:
- WO-A-2007/140621
- WO-A-2008/153377
- THIBAULT H ET AL: "EFFECTS OF LONG-TERM CONSUMPTION OF A FERMENTED INFANT FORMULA (WITH BIFIDOBACTERIUM BREVE C50 AND STREPTOCOCCUS THERMOPHILUS 065) ON ACUTE DIARRHEA IN HEALTHY INFANTS" JOURNAL OF PEDIATRIC GASTROENTEROLOGY AND NUTRITION, RAVEN PRESS, NEW YORK, NY, US, vol. 39, no. 2, 1 August 2004 (2004-08-01) , pages 147-152, XP009043383 ISSN: 0277-2116
- SENOK A C ET AL: 'Probiotics: facts and myths' CLINICAL MICROBIOLOGY AND INFECTION : THE OFFICIAL PUBLICATION OF THE EUROPEAN SOCIETY OF CLINICAL MICROBIOLOGY AND INFECTIOUS DISEASES DEC 2005 LNKD- PUBMED:16307549 vol. 11, no. 12, 01 December 2005, pages 958 - 966, XP002621111

## Description

### Field of the Invention

This invention relates to the prevention and treatment of rotavirus diarrhoea, particularly in infants and small children.

### Background of the Invention

Diarrhoeal diseases remain a major global threat to survival for infants and children and infection with rotavirus is the predominant cause of severe, de-hydrating gastroenteritis in this population in both developing and industrialised countries. In the Western world, the cost of management of rotavirus infection, which has been estimated at over $1 billion per annum in the US alone, is a major drain on increasingly burdened healthcare budgets. The recent development of two new rotavirus vaccines offers hope but, even if an effective vaccine becomes available, its use may be limited by financial constraints in developing countries. Moreover, its efficacy in children with malnutrition and associated immuno-deficiencies is questionable.

In the last few decades, the use of probiotic bacteria has gained considerable attention as a safe and accessible form of treatment for gastrointestinal diseases. Bacteria that have been employed for intervention in cases of diarrhoea of viral origin belong to the genera *Lactobacillus* and *Bifidobacterium*. The therapeutic capacity of certain probiotic bacteria against gastroenteritis of rotaviral origin has been suggested to be due to their ability to stabilise and reinforce the mucosal barrier, production of antimicrobial substances and stimulation of the local antigen-specific and non-specific immune responses. Significant differences have also been noted with regard to the effectiveness and mode of action of different strains.

For example, in the early 1990's, Saavedra *et al* observed that administration of a combination of Bifidobacterium lactis and Streptococcus thermophilus reduced the incidence of diarrhoea and rotavirus shedding in 29 children followed over 18 months in a chronic care ward in a US hospital as compared with 26 children who did not receive any probiotic (Saavedra et al, The Lancet 344, 1046;1994). However, less clear results have been obtained in other trials using Bifidobacteria. For example, a prevention study in 1000 French children showed no decrease in incidence or duration of diarrhoea (Thibault et al, J Ped Gastro Nutr 39, 147;2004).

More recently, WO01/10453 describes the results of an extensive screening of 260 different bacterial strains of which only 4 were shown to essentially inhibit replication of rotavirus. All of the four strains were found to belong to the species *B. longum* or *B adolescents* from which the inventors concluded that these species are particularly effective in the prevention and treatment of rotavirus diarrhoea. A specific strain of *B adolescentis, B adolescents* CNCM I-2168 is particularly highlighted for its ability to prevent infection of human cells by rotaviruses. This strain, however, is stated to have been isolated from adult faeces rendering it less suitable for inclusion in a product designed for infants.

From the foregoing, it may be seen that there remains a need for further probiotics with effective anti-rotavirus activity and which are suitable for incorporation into products for consumption by infants and young children.

### Summary of the Invention

The present inventors have surprisingly found that, contrary to what had previously been thought, anti-rotavirus activity of probiotic *Bifidobacteria* is not something that can be predicted on the basis of species. More specifically, the present inventors have discovered that a particular strain of *Bifidobacterium breve* originally isolated from human milk , *B. breve* CNCM 1-3865, is highly effective in the prevention and treatment of rotavirus diarrhoea, retaining such activity even when in a non-replicating form.

Accordingly, in a first aspect, the present invention provides *Bifidobacterium breve* CNCM I-3865.

In a second aspect, the present invention provides a composition suitable for use in the prevention or treatment of rotavirus diarrhoea comprising *Bifidobacterium brave* CNCM I-3865.

In a third aspect, the present invention provides the use of *Bifidobacterium breve* CNCM I-3865 in the manufacture of a medicament or therapeutic nutritional composition for the prevention or treatment of rotavirus diarrhoea.

In a fourth aspect, the present invention provides a method of prevention or treatment of rotavirus diarrhoea comprising administering to an infant or young child in need thereof a therapeutic amount of *Bifidobacterium breve* CNCM I-3865.

It is known that the intestinal microbiota of healthy, vaginally-delivered, breast-fed infants of age 2 to 4 weeks which may be taken as the optimum microbiota for this age group is dominated by *Bifidobacteria* species whereas formula-fed infants have more complex microbiota, with *Bifidobacteria, Bacteroides, Clostridia* and *Streptococci* all usually present. It had been hypothesised that because breast-fed infants suffered fewer diarrhoeal infections than formula-fed infants, the *Bifidobacteria*-dominant microbiota must confer a protective effect against rotavirus infection. However, a series of studies conducted by the US Centers for Disease Control in Bangladesh did not demonstrate that breast-fed infants suffered fewer instances of rotavirus infections than formula-fed infants (Glass et al, Acta Paediatr. Scand. 75, 713 - 718;1986). Thus, epidemiological data does not support a protective effect of Bifidobacteria in general against rotavirus diarrhoea. The present inventors have demonstrated that although anti-rotavirus activity is found in several members of the genus Bifidobacterium, it is not a species-specific characteristic. This suggests that the genes mediating the anti-rotavirus properties cannot belong to the shared core genome part of the species although no mechanism for the observed effects has yet been elucidated.

### Brief Description of the Drawings

Figure 1 compares the extent of diarrhoea symptoms over time in four groups of mice infected with simian rotavirus and receiving different probiotic interventions;
Figure 2 compares the extent of diarrhoea symptoms over time in three groups of mice infected with simian rotavirus and receiving interventions with the same probiotic in untreated and heat-treated form.

### Detailed Description of the Invention

In this specification, the following terms have the following meanings:-
"infant" means a child under the age of 12 months;
"probiotic" means microbial cell preparations or components of microbial cells with a beneficial effect on the health and well-being of the host. (Salminen S, Ouwehand A. Benno Y. et al "Probiotics: how should they be defined" Trends Food Sci. Technol. 1999:10 107-10);
"young child" means a child between the age of one and six years.

All percentages are by weight unless otherwise stated.

The probiotic *Bifidobacterium breve* CNCM I-3865 may be administered to the infant or young child as a medicament, for example as a daily dose equivalent to 10e10 cfu dissolved in water and administered on a spoon. Alternatively, it may be conveniently administered in an infant formula, a follow-on formula or a growing up milk in an amount equivalent to between 10e3 and 10e12 cfu/g (dry weight basis), more preferably between 10e7 and 10e12 cfu/g. The expression "amount equivalent to" includes the possibilities that the bacteria are live, non-replicating or dead or even present as fragments such as DNA or cell wall materials. In other words, the quantity of bacteria is expressed in terms of the colony forming ability of that quantity of bacteria as if all the bacteria were live irrespective of whether they are, in fact, live, non-replicating or dead, fragmented or a mixture of any or all of these states.

An infant formula according to the present invention may contain a protein source in an amount of not more than 2.0 g/100kcal, preferably 1.8 to 2.0 g/100kcal. The type of protein is not believed to be critical to the present invention provided that the minimum requirements for essential amino acid content are met and satisfactory growth is ensured although it is preferred that over 50% by weight of the protein source is whey. Thus, protein sources based on whey, casein and mixtures thereof may be used as well as protein sources based on soy. As far as whey proteins are concerned, the protein source may be based on acid whey or sweet whey or mixtures thereof and may include alpha-lactalbumin and beta-lactoglobulin in whatever proportions are desired.

The proteins may be intact or hydrolysed or a mixture of intact and hydrolysed proteins. It may be desirable to supply partially hydrolysed proteins (degree of hydrolysis between 2 and 20%), for example for infants believed to be at risk of developing cows' milk allergy. If hydrolysed proteins are required, the hydrolysis process may be carried out as desired and as is known in the art. For example, a whey protein hydrolysate may be prepared by enzymatically hydrolysing the whey fraction in one or more steps. If the whey fraction used as the starting material is substantially lactose free, it is found that the protein suffers much less lysine blockage during the hydrolysis process. This enables the extent of lysine blockage to be reduced from about 15% by weight of total lysine to less than about 10% by weight of lysine; for example about 7% by weight of lysine which greatly improves the nutritional quality of the protein source.

The infant formula may contain a carbohydrate source. Any carbohydrate source conventionally found in infant formulae such as lactose, saccharose, maltodextrin, starch and mixtures thereof may be used although the preferred source of carbohydrates is lactose. Preferably the carbohydrate sources contribute between 35 and 65% of the total energy of the formula.

The infant formula may contain a source of lipids. The lipid source may be any lipid or fat which is suitable for use in infant formulas. Preferred fat sources include palm olein, high oleic sunflower oil and high oleic safflower oil. The essential fatty acids linoleic and α-linolenic acid may also be added as may small amounts of oils containing high quantities of preformed arachidonic acid and docosahexaenoic acid such as fish oils or microbial oils. In total, the fat content is preferably such as to contribute between 30 to 55% of the total energy of the formula. The fat source preferably has a ratio of n-6 to n-3 fatty acids of about 5:1 to about 15:1; for example about 8:1 to about 10:1.

The infant formula may also contain all vitamins and minerals understood to be essential in the daily diet and in nutritionally significant amounts. Minimum requirements have been established for certain vitamins and minerals. Examples of minerals, vitamins and other nutrients optionally present in the infant formula include vitamin A, vitamin B1, vitamin B2, vitamin B6, vitamin B12, vitamin E, vitamin K, vitamin C, vitamin D, folic acid, inositol, niacin, biotin, pantothenic acid, choline, calcium, phosphorous, iodine, iron, magnesium, copper, zinc, manganese, chloride, potassium, sodium, selenium, chromium, molybdenum, taurine, and L-carnitine. Minerals are usually added in salt form. The presence and amounts of specific minerals and other vitamins will vary depending on the intended infant population.

If necessary, the infant formula may contain emulsifiers and stabilisers such as soy lecithin, citric acid esters of mono- and di-glycerides, and the like.

The infant formula may optionally contain other substances which may have a beneficial effect such as fibres, lactoferrin, nucleotides, nucleosides, and the like.

The infant formula described above may be prepared in any suitable manner. For example, they may be prepared by blending together the protein, the carbohydrate source, and the fat source in appropriate proportions. If used, the emulsifiers may be included at this point. The vitamins and minerals may be added at this point but are usually added later to avoid thermal degradation. Any lipophilic vitamins, emulsifiers and the like may be dissolved into the fat source prior to blending. Water, preferably water which has been subjected to reverse osmosis, may then be mixed in to form a liquid mixture. The temperature of the water is conveniently about 50°C to about 80°C to aid dispersal of the ingredients. Commercially available liquefiers may be used to form the liquid mixture. The liquid mixture is then homogenised; for example in two stages.

The liquid mixture may then be thermally treated to reduce bacterial loads, by rapidly heating the liquid mixture to a temperature in the range of about 80°C to about 150°C for about 5 seconds to about 5 minutes, for example. This may be carried out by steam injection, autoclave or by heat exchanger; for example a plate heat exchanger.

Then, the liquid mixture may be cooled to about 60°C to about 85°C; for example by flash cooling. The liquid mixture may then be again homogenised; for example in two stages at about 10 MPa to about 30 MPa in the first stage and about 2 MPa to about 10 MPa in the second stage. The homogenised mixture may then be further cooled to add any heat sensitive components; such as vitamins and minerals. The pH and solids content of the homogenised mixture are conveniently adjusted at this point.

The homogenised mixture is transferred to a suitable drying apparatus such as a spray drier or freeze drier and converted to powder. The powder should have a moisture content of less than about 5% by weight.

The *Bifidobacterium breve* CNCM I-3865 may be cultured according to any suitable method and prepared for addition to the infant formula by freeze-drying or spray-drying for example.

The invention will now be further illustrated by reference to the following examples:-

### Example 1

An example of the composition of an infant formula according to the present invention is given below. This composition is given by way of illustration only.

| Nutrient | per 100kcal | per litre |
|---|---|---|
| Energy (kcal) | 100 | 670 |
| Protein (g) | 1.83 | 12.3 |
| Fat (g) | 5.3 | 35.7 |
| Linoleic acid (g) | 0.79 | 5.3 |
| α-Linolenic acid (mg) | 101 | 675 |
| Lactose (g) | 11.2 | 74.7 |
| Prebiotic (70%FOS, 30% inulin) (g) | 0.64 | 4.3 |
| Minerals (g) | 0.37 | 2.5 |
| Na (mg) | 23 | 150 |
| K (mg) | 89 | 590 |
| Cl (mg) | 64 | 430 |
| Ca (mg) | 62 | 410 |
| P(mg) | 31 | 210 |
| Mg (mg) | 7 | 50 |
| Mn (µg) | 8 | 50 |
| Se (µg) | 2 | 13 |
| Vitamin A (µg RE) | 105 | 700 |
| Vitamin D (µg) | 1.5 | 10 |
| Vitamin E (mg TE) | 0.8 | 5.4 |
| Vitamin K1 (µg) | 8 | 54 |
| Vitamin C (mg) | 10 | 67 |
| Vitamin B1 (mg) | 0.07 | 0.47 |
| Vitamin B2 (mg) | 0.15 | 1.0 |
| Niacin (mg) | 1 | 6.7 |
| Vitamin B6 (mg) | 0.075 | 0.50 |
| Folic acid (µg) | 9 | 60 |
| Pantothenic acid (mg) | 0.45 | 3 |
| Vitamin B 12 (µg) | 0.3 | 2 |
| Biotin (µg) | 2.2 | 15 |
| Choline (mg) | 10 | 67 |
| Fe (mg) | 1.2 | 8 |
| I (µg) | 15 | 100 |
| Cu (mg) | 0.06 | 0.4 |
| Zn (mg) | 0.75 | 5 |
| *Bifidobacterium breve* CNCM I-3865 | 2.10⁷ cfu/g of powder | |

### Example 2

This example compares the efficacy of three different strains of *B. breve* against rotavirus infection in mice. The particular mouse model was chosen for three reasons. First, in this model simian rotavirus not only caused intestinal rotavirus infection but also rotavirus diarrhoea. Second, the histopathology of the rotavirus effect on the murine intestinal mucosa resembles that described for rotavirus infected children. Third, this model has reproduced the greater and lesser effects achieved with different probiotic strains in clinical trials.

14 days pregnant BALB/c mice were purchased from Mollegard, Denmark. The mice were housed individually in the animal facility at the Karolinska University Hospital, Huddinge, Sweden. Bedding and nesting material, normal pellet diet and water were provided *ad libitum*. A 12:12 hours light:dark cycle was maintained. Pups were born at 19 to 20 days' gestation. Pups from a litter were randomised before being redistributed to different experimental groups with 7 to 10 pups per group. Four groups of four day old pups were used for the study (three experimental groups and one control group). Bacteria were administered to the pups once daily in a 10 µl volume starting on Day-1 and continuing until Day 3. Freshly cultured bacteria were resuspended in PBS at a concentration of 10e10 cfu/ml (the PBS medium had previously been tested in the same animal model and was not found to influence the diarrhoea outcome). The three experimental groups received, respectively *B. breve* CNCM I-3865 (also designated by the internal reference NCC 2950), *B. breve* NCC 2791 and *B breve* NCC 458. The control group received only the PBS medium.

Infection was induced orally on Day 0 using 2.10e7 FFU simian rotavirus RRV. A single batch of RRV was used for the whole study. Occurrence of diarrhoea was recorded daily until Day 4. Pups were euthanized on Day 4 using intra-peritoneal pentobarbital.

Diarrhoea in the pups was assessed on the basis of consistency of faeces. Watery diarrhoea was given a score of 2, loose stools a score of 1 and no stools or normal stools a score of 0. Presence or absence of diarrhoea was compared between treatment groups on a daily basis by Fisher's exact test and was presented as percentage diarrhoea in graphs. Severity was defined as the sum of diarrhoea scores for each pup during the course of the experiment (severity = X diarrhoea score (Day 1 + Day 2 + Day 3 + Day 4)) and duration was defined as the total sum of days with diarrhoea. Both severity and duration were analysed by Kruskal-Wallis and Dunn tests. Differences in the intestinal virus load as assessed by real time PCR were tested using the Mann-Whitney test.

The infection rate, severity and duration are shown in Table 2 below.

**Table 2**

| | NCC 2950 | NCC 2791 | NCC 458 | Control |
|---|---|---|---|---|
| No of values | 8 | 8 | 8 | 6 |
| Mean duration | 0.75 | 1.5 | 2.125 | 2.5 |
| SE duration | 0.2500 | 0.2673 | 0.2266 | 0.5000 |
| Mean severity | 1 | 1.75 | 3 | 3.667 |

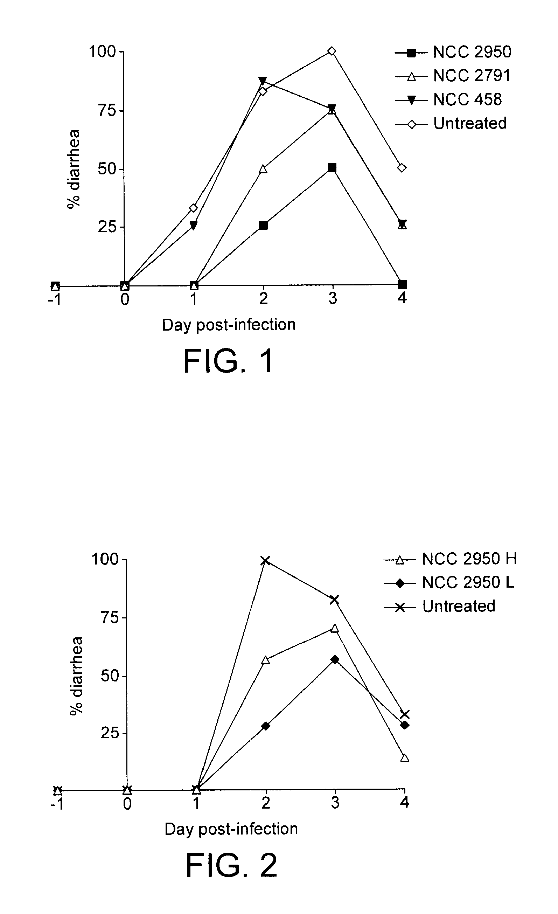

As may be seen from Table 2 and Figure 1, a high infection rate was achieved in the control animals where diarrhoea occurred in 100% of the subjects at day 3, thus allowing a robust statistical analysis. *B. breve* CNCM I-3865 showed a significant reduction both with respect to diarrhoea duration and diarrhoea symptom score (Figure 1). By contrast *B. breve* NCC 2791 showed a limited protective effect whilst the results from the *B. breve* NCC 458 resembled those in the control group.

### Example 3

This example compares the efficacy of two different preparations of *B. breve* CNCM I-3865 against rotavirus infection in mice using the model described above in Example 2. In this example, there were two experimental groups and a control group. One experimental group received live bacteria resuspended in PBS at 10e10 cfu/ml (NCC 2950 L). The other experimental group received a similar bacterial preparation except that the preparation was heat-treated at 90°C for 30 minutes prior to administration (NCC 2950 H). The control group received only the PBS medium. The results are shown in Table 3 and Figure 2. It may be seen that a high infection rate was achieved in the control group where 100% of the animals had diarrhoea at day 2. Both experimental groups had reduced symptoms of diarrhoea when compared to the control group.

**Table 3**

| | NCC 2950 H | NCC 2950 L | Control |
|---|---|---|---|
| No of values | 7 | 7 | 6 |
| Mean duration | 1.429 | 1.143 | 2.167 |
| SE duration | 0.2974 | 0.4592 | 0.3073 |
| Mean severity | 1.429 | 1.429 | 4 |

## Claims

1. *Bifidobacterium breve* CNCM 1-3865.

2. A composition suitable for use in the prevention or treatment of rotavirus diarrhoea comprising *Bifidobacterium breve* CNCM T-3865.

3. A composition according to Claim 2 which is an infant formula, a follow-on formula or a growing-up milk.

4. A composition according to Claim 3 which comprises *Bifidobacterium breve* CNCM 1-3865 in an amount equivalent to between 10e3 and 10e12 cfu/g on a dry weight basis.

5. A composition according to Claim 3 or 4 which comprises *Bifidobacterium breve* CNCM I-3865 in an amount equivalent between 10e7 and 10e12 cfu/g.

6. A composition according to any of Claims 3 to 5 which further comprises at least one prebiotic in an amount of from 0.3 to 6% by weight of the composition.

7. A composition according to any of Claim 2 which is a supplement and which comprises from 10⁴ to 10¹² cfu of *Bifidobacterium breve* CNCM I-3865 per unit dose.

8. Use of a composition as claimed in any one of claims 1 to 7 in the manufacture of a medicament or therapeutic nutritional composition for the prevention or treatment of rotavirus diarrhoea.

## Patentansprüche

1. *Bifidobacterium breve* CNCM I-3865.

2. Zusammensetzung, die für die Verwendung bei der Prävention oder Behandlung von Rotavirus-Diarrhöe, aufweisend *Bifidobacterium breve* CNCM I-3865, geeignet ist.

3. Zusammensetzung nach Anspruch 2, die eine Babynahrung, eine Folgenahrung oder eine Milch für Kleinkinder ist.

4. Zusammensetzung nach Anspruch 3, die *Bifidobacterium breve* CNCM I-3865 in einer Menge zwischen 10e3 und 10e12 KbE/g in der Trockenmasse aufweist.

5. Zusammensetzung nach Anspruch 3 oder 4, die *Bifidobacterium breve* CNCM I-3865 in einer Menge zwischen 10e7 und 10e12 KbE/g aufweist.

6. Zusammensetzung nach einem der Ansprüche 3 bis 5, die weiterhin mindestens ein Präbiotikum in einer Menge von 0,3 bis 6 Gew.-% der Zusammensetzung aufweist.

7. Zusammensetzung nach Anspruch 2, die eine Nahrungsergänzung ist, und die von 10⁴ bis 10¹² KbE von *Bifidobacterium breve* CNCM I-3865 je Einzeldosis aufweist.

8. Verwendung einer Zusammensetzung nach einem der Ansprüche 1 bis 7 bei der Herstellung eines Medikamentes oder einer therapeutischen Nahrungszusammensetzung zur Prävention oder Behandlung von Rotavirus-Diarrhöe.

## Revendications

1. *Bifidobacterium breve* CNCM I-3865.

2. Composition appropriée pour une utilisation dans la prévention ou le traitement de la diarrhée à rotavirus comprenant *Bifidobacterium breve* CNCM I-3865.

3. Composition selon la revendication 2, qui est une préparation pour nourrisson, une préparation de suite ou un lait de croissance.

4. Composition selon la revendication 3, qui comprend *Bifidobacterium breve* CNCM I-3865 dans une quantité équivalente à entre 10e3 et 10e12 ufc/g sur la base du poids sec.

5. Composition selon la revendication 3 ou 4, qui comprend *Bifidobacterium breve* CNCM I-3865 dans une quantité équivalente entre 10e7 et 10e12 ufc/g.

6. Composition selon l'une quelconque des revendications 3 à 5, qui comprend en outre au moins un prébiotique dans une quantité allant de 0,3 à 6% en poids de la composition.

7. Composition selon la revendication 2, qui est un supplément et qui comprend de 10⁴ à 10¹² ufc de *Bifidobacterium breve* CNCM I-3865 par dose unitaire.

8. Utilisation d'une composition selon l'une quelconque des revendications 1 à 7 dans la fabrication d'un médicament ou d'une composition nutritionnelle thérapeutique pour la prévention ou le traitement de la diarrhée à rotavirus.
